# EUROPEAN PATENT APPLICATION

(11) **EP 2 266 550 A1**
(43) Date of publication of application: **29.12.2010**
(21) Application number: 09305542.4
(22) Date of filing: 15.06.2009
(51) Int. Cl.: A61K 31/00, A61P 25/14, A61P 25/16, A61P 25/28, A61K 38/39, A61K 31/07, A61K 31/12, A61K 31/165, A61K 31/192, A61K 31/365, A61K 31/405, A61K 31/415, A61K 31/506, A61K 31/593, A61K 31/60, A61K 31/7088, A61K 33/00, A61K 38/17, A61K 39/395, A61K 48/00, A61K 45/06, G01N 33/68

(54) **Antagonists of ß-catenin for preventing and/or treating neurodegenerative disorders**

(71) Applicant: Institut Curie, 75248 Paris Cedex 05 (FR); Centre National de la Recherche Scientifique (CNRS), 75116 Paris (FR)
(72) Inventor: Humbert, Sandrine, 91400 ORSAY (FR); Saudou, Frèdèric, 91400 ORSAY (FR); Godin, Juliette, 92120 Montrouge (FR)
(74) Representative: Jacobson, Claude

(57) **Abstract**

The invention relates to antagonists of β-catenin for use in the prevention and/or treatment of neurodegenerative disorders. β-catenin has been found to specifically accumulate in Huntington's disease and this accumulation is toxic in striatal neuron expressing mutant huntingtin, the causal protein of Huntington's disease. Since β-catenin had been previously reported to accumulate in Alzheimer's disease, interfering with β-catenin accumulation in neuronal cells provides a therapeutic approach for the prevention and/or treatment of neurodegenerative disorders.

## Description

The invention relates to antagonists of β-catenin for use in the prevention and/or treatment of neurodegenerative disorders. β-catenin has been found to specifically accumulate in Huntington's disease and this accumulation is toxic in striatal neurons expressing mutant huntingtin, the causal protein of Huntington's disease. Since β-catenin had been previously reported to accumulate in Alzheimer's disease, interfering with β-catenin accumulation in neuronal cells provides a therapeutic approach for the prevention and/or treatment of neurodegenerative disorders.

Huntington's disease (HD) is a devastating neurodegenerative disorder which is dominantly inherited and is characterized by choreiform movements, psychiatric and cognitive decline, and the graded loss of medium spiny projection neurons in the striatum and of cortical neurons. HD is initiated by an unstable CAG triplet repeat that lengthens a polyglutamine tract in the huntingtin (Htt) protein above ∼37 residues, such that disease severity is increased with increased polyglutamine length. The expanded polyglutamine (poly(Q)) tract in Htt protein (polyQ-Htt) confers a novel gain of function on the mutant protein. Disease symptoms typically manifest in mid-life caused by mutant huntingtin with about 50 glutamines or in juvenile years caused by mutant huntingtin with more than ∼55 glutamines. The pathogenic mechanisms that lead to disease are not fully understood but a pathological hallmark of HD is the formation of neuronal intracellular inclusions (Nlls) caused by aggregates of polyQ-Htt. These inclusions are also found in other triplet diseases, and in particular polyglutamine diseases. Although polyQ-Htt aggregates are apparently not directly associated with apoptosis induction, the aggregates would induce neuronal dysfunction, such as proteasome activity alteration. There is currently no treatment to delay or prevent death of the neurons and appearance or progression of the symptoms.

As of today, ten different neurodegenerative disorders caused by polyglutamine expanded tract have been identified. Although the genes involved in different polyglutamine diseases have little in common, the disorders they cause follow a strikingly similar course. Each disease is characterized by a progressive degeneration of a distinct group of nerve cells. The major symptoms of these diseases are similar, although not identical, and usually affect people in midlife.

The Inventors have found that β-catenin specifically accumulates in HD, as identified in various cell types that express polyQ-Htt, in *Drosophila* models of HD and in post-mortem samples from patients. They have further demonstrated that accumulation of β-catenin is toxic in striatal neurons expressing polyQ-Htt as reducing β-catenin levels is protective.

Divergent findings were reported as regards involvement of β-catenin in Huntington's disease.

A comprehensive study of huntingtin protein interactors by high-throughput yeast two-hybrid screening and affinity pull down followed by mass spectrometry led to the identification of 234 high-confidence Htt-associated proteins, including β-catenin (Kaltenbach et al. (2007). PLoS Genet 3(5): e82). An arbitrary set of 60 proteins was further selected to assay ability to modify a neurodegenerative phenotype in *Drosophila* induced by a 336 amino acid long Htt-fragment. 80% of the genes enhanced or suppressed the neurodegenerative phenotype induced in *Drosophila,* 45% (27) of the genes assayed being regarded as high-confidence modifiers. 18 of these 27 genes (i.e. 30% of the overall genes assayed) behaved as suppressors of the neurodegenerative phenotype, either by partial loss-of-function or by over-expression. Within these 18 genes, reduced levels of armadillo, the *Drosophila* homologue of β-catenin, were reported to suppress the Htt-induced phenotype.

It had been previously demonstrated in genetic Huntington's disease mouse and striatal cell models that early events of the disease are associated with activation of the Akt pro-survival signaling pathway. Akt activation in mutant striatal cells was further found to be associated with inactivation of GSK3β (glycogen synthase kinase 3 β), via phosphorylation of GSK3β at Ser9, resulting in increased β-catenin stabilization and cytosolic concentration. β-catenin is an essential transcriptional coactivator whose turnover and translocation to the nucleus is regulated by GSK3β phosphorylation. However, this increased β-catenin cytosolic concentration was regarded as a pro-survival response, as enhanced β-catenin signalling was assumed to be protective from the effect of mutant hungtintin (Gines et al. (2003) J Biol Chem 278(50): 50514-22).

Furthermore, it was found that lithium treatment (LiCI) in Huntington's disease cell models reduces poly(Q) toxicity. This effect was associated with GSK-3β inhibition as poly(Q) toxicity was also reduced by SB216763, a GSK-3β inhibitor. GSK-3β inhibition by LiCl and SB216763 resulted in increased β-catenin dependent T-cell factor mediated transcription. β-catenin levels were actually found to be reduced in cells expressing expanded polyQ repeats (Carmichael et al. (2002) J Biol Chem. 277(37):33791-8).

Therefore, β-catenin levels have been found to be both increased and, conversely, decreased in cell and animal models of Huntington's disease. Additionally, although a partial loss-of-function mutation in armadillo (β-catenin) was described as being associated with suppression of neurodegenerative phenotype in a *Drosophila* model of Huntington's disease, accumulation of β-catenin was generally assumed to represent a protective response in the context of Huntington's disease.

Normally cytopasmic β-catenin level is kept low through continuous ubiquitin-proteasome-mediated degradation of β-catenin, which is regulated by a so-called "destruction complex". In this complex, Axin and adenomatous polyposis coli (APC) function as scaffold proteins that facilitate sequential phosphorylation of β-catenin at the N-terminus by casein kinase lα (CKIα; on Ser45) and GSK3β (on Ser33, Ser37 and Thr41). Phosphorylated β-catenin is then recognized by β-TrCP (β-Transducing repeat containing protein), a component of the E3 ubiquitin ligase complex, and targeted for ubiquitin-mediated proteosomal degradation.

The Inventors investigated the mechanisms by which mutant huntingtin leads to cytoplasmic β-catenin accumulation by examining whether huntingtin could interact with and regulate the destruction complex. It was found that polyQ-huntingtin leads to the stabilization of β-catenin by reducing its interaction with the destruction complex and β-TrCP.

The Inventors further demonstrated that manipulating this pathway regulates mutant-htt-induced toxicity. In particular, overexpression of β-TrCP exerts a neuroprotective effect in a neuronal model of HD which depends on β-TrCP ability to bind the E3 ubiquitin ligase complex, SCF, as a mutant form of β-TrCP that cannot bind SCF is not protective. Moreover, chimeric Fbox proteins that can only degrade β-catenin had no effect on neuronal toxicity by themselves, but significantly reduced polyQ-induced toxicity to levels comparable to the wild-type situation indicating that the neuroprotective properties of β-TrCP in HD specifically depend on its capacity to degrade β-catenin. Together these results indicate that pathways or compounds that reduce β-catenin accumulation are of therapeutic interest for HD.

This was further confirmed *in vivo* in a relevant validated *Drosophila* model of HD. The toxic accumulation of β-catenin could be rescued by the overexpression of β-TrCP in HD flies. Indomethacin, an inhibitor of cyclooxygenase which was shown to downregulate β-catenin mRNA and protein level in cells, was further assayed in a neuronal model of HD and in the *Drosophila* model of HD. It was observed that indomethacin treatment also reduced the level of β-catenin in neurons, and the eye depigmentation phenotype observed in HD flies was completely abolished by the treatment. It was thus demonstrated that indomethacin rescues degeneration in cells and that this drug could be a suitable HD treatment.

Altogether, it is proposed that Htt could be a scaffold protein which facilitates β-catenin interaction with β-TrCP and association with the destruction complex. In HD context, association with the destruction complex would be altered due to the loss of β-catenin / β-TrCP interaction, leading to a cytoplasmic accumulation of β-catenin. This model is in agreement with results showing weak binding of β-catenin to β-TrCP suggesting that other proteins may participate in stabilization of this interaction (Wu et al. (2003) Mol Cell. 11 (6):1445-56).

More generally, these results support that antagonists of β-catenin represent a possible therapeutic approach for the treatment of Huntington's disease and polyglutamine diseases.

Furthermore, increased β-catenin levels were also found in Alzheimer's disease. More specifically, it was shown that presenilin 1 (PS1) deficiency, one of the major causes of Alzheimer's disease onset, results in elevated cytosolic β-catenin levels suggesting that PS1 could be involved in targeting phosphorylated β-catenin for degradation (Soriano et al. (2001) J Cell Biol. 152(4):785-94). Presenilin 1 was indeed further demonstrated to contribute to β-catenin degradation by facilitating the stepwise phosphorylation of β-catenin independently from the Axin destruction complex (Kang et al. (2002) Cell. 110(6):751-62).

Accordingly, huntingtin and presenilin 1 are both examples of proteins contributing to neurodegenerative disease onset which are found to be implicated in β-catenin degradation in wild-type conditions.

It is thus proposed that impaired β-catenin degradation, and resulting increased β-catenin signalling, could be a general mechanism leading to neuron dysfunction in neurodegenerative diseases.

### Methods of treatment

The invention thus relates to an antagonist of β-catenin for use for preventing and/or treating a neurodegenerative disorder.

The invention also relates to an antagonist of β-catenin for the manufacture of a medicament intended for preventing and/or treating a neurodegenerative disorder.

The invention further provides for a method of preventing and/or treating a neurodegenerative disorder which comprises administering a patient in need thereof with an antagonist of β-catenin.

In particular, the antagonist of β-catenin is intended to interfere with cytoplasmic accumulation of β-catenin in a neuronal cell.

As used herein, the term "subject" or "patient" denotes a human or non-human mammal, such as a rodent, a feline, a canine, or a primate.

The prevention and/or treatment of a neurodegenerative disorder in a human patient is preferably contemplated.

In the context of the invention, the term "treating" or "treatment" is used herein to characterize a method or process that is aimed at (1) delaying or preventing the onset of a disorder or condition to which such term applies; (2) slowing down or stopping the progression, aggravation, or deterioration of the symptoms of the disease state or condition to which such term applies; (3) alleviating or bringing about ameliorations of the symptoms of the disease state or condition to which such term applies; and/or (4) reversing or curing the disease state or condition to which such term applies. A treatment may be administered prior to the onset of the disease, for a prophylactic or preventive action. Alternatively or additionally, a treatment may be administered after initiation of the disease or condition, for a therapeutic action.

The neurodegenerative disorder may be selected from the group consisting of polyglutamine (polyQ) diseases, Alzheimer's disease, Parkinson's disease, prion diseases, multiple sclerosis, and amyotrophic lateral sclerosis.

Preferably the neurodegenerative disorder is a disorder in which neuronal toxicity is associated with cytoplasmic β-catenin accumulation, in particular due to impaired β-catenin degradation.

Prion diseases are also called "spongiform encephalopathies" and include Creutzfeld-Jacob Disease, Gerstmann-Straussler-Scheinker syndrome, Fatal familial Insomnia, Kuru, Alpers Syndrome, bovine spongiform encephalopathy, scrapie, transmissible mink encephalopathy and chronic wasting disease.

Preferably, the neurodegenerative disorder is a polyglutamine (polyQ) disease selected from the group consisting of Hungtinton's disease, Kennedy disease, Haw River syndrome, Spinocerebellar ataxia type 1, Spinocerebellar ataxia type 2, Spinocerebellar ataxia type 3, Spinocerebellar ataxia type 6, Spinocerebellar ataxia type 7, Spinocerebellar ataxia type 12 and Spinocerebellar ataxia type 17.

Still preferably, the neurodegenerative disorder is Hungtinton's disease.

The genes involved in the different polyglutamine diseases and the numbers of glutamine repeat usually recognized as associated with onset of the disease are reported in Table 1.

Table 1 below identifies the causative proteins implicated in these polyglutamine diseases.

**Table 1: polyglutamine diseases**

| **Disease** | **Gene name** | **Chromosomal location** | **Pattern of inheritance** | **Protein** | **Normal repeat length** | **Disease repeat length** |
|---|---|---|---|---|---|---|
| Spinobulbar muscular atrophy (Kennedy disease) | *AR* | Xq13-21 | X-linked recessive | androgen receptor (AR) | 11-33 | 40-62 |
| Huntington disease | *HD* | 4p16.3 | autosomal dominant | huntingtin | 9-34 | 35-240 |
| Dentatorubral-pallidoluysian atrophy (Haw River syndrome) | *DRPLA* | 12p13.31 | autosomal dominant | atrophin-1 | 7-23 | 49-75 |
| Spinocerebellar ataxia type 1 | *SCA1* | 6p23 | autosomal dominant | ataxin-1 | 6-44 | 39-83 |
| Spinocerebellar ataxia type 2 | *SCA2* | 12q24.1 | autosomal dominant | ataxin-2 | 14-31 | 33-64 |
| Spinocerebellar ataxia type 3 (Machado-Joseph disease) | *SCA3* | 14q32.1 | autosomal dominant | ataxin-3 | 12-40 | 54-86 |
| Spinocerebellar ataxia type 6 | *SCA6* | 19p13 | autosomal dominant | a1_{A}-voltage-dependent calcium channel subunit | 4-20 | 20-31 |
| Spinocerebellar ataxia type 7 | *SCA7* | 3p12-13 | autosomal dominant | ataxin-7 | 4-27 | 37->200 |
| Spinocerebellar ataxia type 12 | *SCA12* | 5q31-33 | autosomal dominant | Protein phosphatase 2 regulatory subunit B | <29 | 66-93 |
| Spinocerebellar ataxia type 17 | *SCA17* | 6q27 | autosomal dominant | TATA binding protein | 25-42 | 45-63 |

### Antagonists of β-catenin

As used herein, an "antagonist of β-catenin" denotes an agent according to the present invention (i.e., molecule) which inhibits or blocks β-catenin activity, in particular in a neuronal cell. The antagonist of β-catenin may inhibit β-catenin expression (i.e. β-catenin gene transcription and/or translation) and/or β-catenin functional activity. Preferably, the antagonist is a direct antagonist of β-catenin.

β-catenin activity is determined by the amount of β-catenin in the cytoplasm. Non-phosphorylated β-catenin escaping β-TrCP recognition and subsequent degradation is translocated into the nucleus where it forms a complex with a transcription factor of the T-cell factor (TCF)/lymphocyte enhancer factor (LEF) family and induces expression of Wtn target genes including c-myc and cyclin D1. Human transcription factors of the TCF/LEF family include TCF1 (also called TCF7), TCF3 (also called TCF7L1), TCF4 (also called TCF7L2), and LEF1.

β-catenin stimulates expression of Wtn target genes by recruiting various co-activators, including CBP (CREB-binding protein) and p300.

The Wtn/ β-catenin pathway has been reviewed for instance by Takemaru et al. (2008, Handb Exp Pharmacol. (186):261-84).

The β-catenin antagonist may inhibit β-catenin expression, preferably in a brain cell.

The β-catenin antagonist may also interfere with cytoplasmic accumulation of β-catenin, preferably in a brain cell. In particular, said β-catenin antagonist may increase β-catenin degradation, especially by the Axin degradation complex. The β-catenin antagonist may also induce β-catenin relocalization at the cell membrane.

Alternatively, the β-catenin antagonist may interferes with β-catenin binding to a transcription factor of the T-cell factor (TCF)/lymphocyte enhancer factor (LEF) family or to a β-catenin co-activator.

The β-catenin antagonists according to the invention are capable of inhibiting or eliminating the functional activity of β-catenin *in vivo* and/or *in vitro.* The antagonist may inhibit the functional activity of β-catenin by at least about 10%, preferably by at least about 30%, preferably by at least about 50%, preferably by at least about 70, 75 or 80%, still preferably by 85, 90, 95, or 100%.

The one skilled in the art may readily assess if a candidate molecule is an antagonist of β-catenin according to known methods.

For instance, it can be assayed whether a candidate molecule decreases expression of β-catenin or increases degradation of β-catenin by qualitatively or quantitatively assaying variation of β-catenin levels in a cell expressing β-catenin which was contacted with said candidate molecule, as compared with levels of β-catenin levels observed in said cell under the same conditions but in the absence of the candidate molecule. β-catenin levels may be determined by any conventional method, such as antibody-based methods like Westen blot, immunoprecipitation, or ELISA.

It can also be assayed whether a candidate molecule interferes with β-catenin binding to TCF/LEF for instance using a TCF/LEF Reporter kit, such as commercially available through SABiosciences. Use may be made of a cell transfected with a TCF/LEF-responsive reporter gene construct which encodes a reporter gene under the control of a minimal CMV promoter and tandem repeats of the TCF/LEF transcriptional response element (TRE). Detection of the signal associated with the expression of the reporter gene is indicative of β-catenin binding with TCF/LEF resulting in activation of TRE. Accordingly, a decrease in, or absence of, reporter gene signal when the cell is contacted with a candidate molecule is indicative of a candidate molecule interferes with β-catenin binding to TCF/LEF.

Alternatively, as screen base on surface Plasmon resonance studies, such as described by Lepourcelet et al. (2004. Cancer Cell. 5(1):91-102), may be used to identify antagonist of β-catenin/TCF interaction.

The above methods are non limitative examples of methods which can be used in the frame of the invention to determine if a candidate molecule is a β-catenin antagonist.

The β-catenin antagonist may be for instance a peptide or polypeptide, such as an antibody directed against β-catenin; a nucleic acid molecule such as an aptamer, a β-catenin anti-sense oligonucleotide, a β-catenin siRNA, or a ribozyme; or a small molecule inhibitor of β-catenin activity

As used herein, "antibody" refers to immunoglobulin molecules and immunologically active portions of immunoglobulin molecules, i.e., molecules that contain an antigen binding site which immunospecifically binds an antigen. As such, the term antibody encompasses not only whole antibody molecules, but also antibody fragments as well as variants (including derivatives) of antibodies and antibody fragments. In particular the antibody may be monoclonal (recombinant or produced by hybridoma), humanized, and/or a fragment such as Fv, Fab, F(ab')₂, Fd, dAb, dsFv, scFv, sc(Fv)₂.

Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by EXponential enrichment (SELEX) of a random sequence library, as described in Tuerk C. and Gold L., Science, 1990, 249(4968):505-10. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., Clin. Chem., 1999, 45(9):1628-50. Peptide aptamers consists of a conformationally constrained antibody variable region displayed by a platform protein, such as E. coli Thioredoxin A that are selected from combinatorial libraries by two hybrid methods (Colas et al., Nature, 1996,380, 548-50).

In particular, the β-catenin antagonist may be an antibody or aptamer directed against β-catenin and which blocks interaction of β-catenin with TCF/LEF.

Antisense oligonucleotides comprise fragments of the targeted polynucleotide sequence encoding β-catenin. Such a fragment generally comprises at least about 14 nucleotides, typically from about 14 to about 30 nucleotides. The ability to derive an antisense oligonucleotide, based upon a nucleic acid sequence encoding a given protein is described in, for example, Stein and Cohen (Cancer Res., 1988, 48:2659), and van der Krol et al. (BioTechniques, 1988, 6:958). Antisense oligonucleotides comprise oligonucleotides having modified sugar-phosphodiester backbones (or other sugar linkages, such as those described in WO 91/06629) and wherein such sugar linkages are resistant to endogenous nucleases. Antisense oligonucleotides may be introduced into a cell containing the target nucleic acid by any gene transfer method, including, for example, electroporation, microinjection, transduction, cell fusion, DEAE dextran, calcium phosphate precipitation, use of a gene gun, or lipofection, or by using gene transfer vectors such as Epstein-Barr virus or adenovirus.

RNA interference (RNAi) produced by the introduction of specific small interfering RNA (siRNA), as described, for example in Bosher et al., Nature Cell Biol 2, E31-E36 (2000). β-catenin duplexed siRNA which may be used in the frame of the invention have already been described by Gu et al. (2008 Mol Cell Biol. 28(16):4963-74).

Ribozymes are enzymatic RNA molecules capable of catalyzing the specific cleavage of RNA. The mechanism of ribozyme action involves sequence specific hybridization of the ribozyme molecule to complementary target RNA, followed by endonucleolytic cleavage. Engineered hairpin or hammerhead motif ribozyme molecules that specifically and efficiently catalyze endonucleolytic cleavage of β-catenin mRNA sequences are thereby useful within the scope of the present invention.

In particular, the antagonist may be selected from the group consisting of non-steroidal antiinflammatory drugs (NSAIDs), inhibitors of cyclooxygenase (COX) enzymes, a Histone deacetylase (HDAC) inhibitor, imatinib or a salt or ester thereof, endostatin, a β-catenin antisense oligonucleotide, a β-catenin siRNA, a vector containing a β-TrCP encoding sequence, a F-box chimera, vitamin A and retinoids, vitamin D, differentiation-inducing factors (DIFs), lithium, curcumin, flavonoids, and small molecule inhibitors of β-catenin binding to TCF/Lef or a β-catenin co-activator.

NSAIDs are widely prescribed for the treatment of pain, fever and inflammation. Their effects are largely attributed to the inhibition of cyclooxygenase COX-1 and/or COX-2.

NSAIDs have also been reported to inhibit the Wnt/β-catenin signaling pathway. For instance indomethacin would repress β-catenin transcription and induce β-catenin protein degradation whereas aspirin (acetyl salicylic acid) would stabilize β-catenin in its inactive phosphorylated form, thereby preventing its function as a co-transcription factor. Nitric oxide releasing aspirin (NO-ASA) is suggested to affect β-catenin signalling by disrupting the β-catenin/TCF complex in the nucleus. Sulindac and sulindac metabolites would induce β-catenin degradation. The mechanisms of action of these NSAIDs on the Wnt/β-catenin pathway have been reviewed by Dihlmann and von Knebel Doeberitz (2005. Int J Cancer. 113(4):515-24). Accordingly, NSAIDs which may be used in the frame of the invention may be for instance indomethacin, sulindac, sulindac metabolite, naproxen, ketoprofen, ibuprofen, acetyl salicylic acid, NO-acetyl salicylic acid, celecoxib, and rofecoxib.

Histone deacetylase (HDAC) inhibitors, in particular HDAC6 inhibitors, may also be used in the frame of the invention as preventing β-catenin deacetylatation on Lys49 render β-catenin liable to phosphorylation on Lys45 and to subsequent degradation by the destruction complex. HDAC inhibitors include for instance suberoylanilide hydroxamic acid (SAHA), trichostatin A (TSA), NaPB, and resveratrol.

Endostatin in known to induce β-catenin degradation by the proteasome.

Glivec® (imatinib mesylate) downregulates β-catenin signalling, probably by inhibiting tyrosine-phosphorylation of β-catenin.

Vitamin A and its metabolites (retinoids), as well as vitamin D, differentiation-inducing factors (DIFs), lithium, curcumin, and flavonoids, have been reported to inhibit the Wnt/ β-catenin pathway (for a review see e.g. Takahashi-Yanaga and Sasaguri (2009) J Pharmacol Sci. 109(2):179-83).

Small molecule inhibitors, such as PKF115-854, CGP049090, and ICG-001, have been reported to disrupt β-catenin/TCF complex (Lepourcelet et al. 2004. Cancer Cell. 5(1):91-102).

A F-box chimeric protein, or F-box chimera, may also be used to achieve very specific degradation of β-catenin. The F-box motif of β-TrCP is known to assign β-catenin for degradation. F-box chimeric proteins may consist in a F-box protein in which the WD40 domain is replaced by TCF4 or E-cadherin binding domain to β-catenin (Cong et al. (2003) BMC Mol Biol. 4:10; Liu et al. (2004) Biochem Biophys Res Commun. 313(4):1023-9) or in a F-box protein fused to 14aa repeats of APC protein (Su et al. (2003) Proc Natl Acad Sci U S A. 100(22):12729-34). These chimeric protein very specifically recognize cytoplasmic and nuclear β-catenin, thereby enhancing specific β-catenin degradation (Su et al. (2003) Proc Natl Acad Sci U S A. 100(22):12729-34, 2003; Liu et al. (2004) Biochem Biophys Res Commun. 313(4):1023-9). Reference is made to the review by Dihlmann and von Knebel Doeberitz (2005) Int J Cancer. 113(4):515-24.

Increased β-catenin degradation may also be obtained by over-expressing β-TrCP, for instance by delivery a vector, such as a virus, containing a β-TrCP encoding sequence.

According to an embodiment, the β-catenin antagonist is elected from the group consisting of indomethacin, sulindac, sulindac metabolite, naproxen, ketoprofen, ibuprofen, acetyl salicylic acid, NO-acetyl salicylic acid, celecoxib, rofecoxib, PKF115-854, CGP049090, ICG-001 and suberoylanilide hydroxamic acid (SAHA), trichostatin A (TSA), and NaPB.

Preferably, the β-catenin antagonist is selected from the group consisting of a β-catenin antisense oligonucleotide, a β-catenin siRNA, an antibody or aptamer directed against β-catenin and which blocks interaction of β-catenin with TCF/LEF, a vector containing a β-TrCP encoding sequence, and a F-box chimera.

### Other active substances

An active substance may be used in combination with said antagonist of β-catenin activity, either by simultaneous administration (e.g. when the antagonist of β-catenin activity and the at least one other active substance are formulated in a same pharmaceutical composition), or sequential administration ((e.g. when the antagonist of β-catenin activity and the at least one other active substance are administered separately in time).

The other active substance may also be a β-catenin antagonist but not necessarily. It may be an active substance which may be used for the prevention and/or treatment of the neurodegenerative disorder intended to be prevented/treated, but which acts by mechanisms not mediated by β-catenin, or not only mediated by β-catenin.

The active substance may be for instance a histone deacetylase (HDAC) inhibitor used in combination another type of β-catenin antagonist.

HDAC6 has been shown to deacetylate β-catenin on Lys49, thereby preventing β-catenin phosphorylation on Lys45 and subsequent degradation by the destruction complex.

Beside this direct effect of HDAC inhibitors on β-catenin activity, recent studies have identified an altered microtubule (MT)-dependent transport of organelles in HD. It was previously demonstrated that htt associates with molecular motors and activates the MT-dependent transport of vesicles containing brain-derived neurotrophic factor (BDNF). Wild-type (WT) htt enhances the velocity of the vesicles and reduces the amount of time they spend not moving (pausing time). In HD, in which the htt contains the polyQ expansion, the intracellular transport of BDNF-containing vesicles is altered, resulting in reduced trophic support of neurons and their death.

Histone deacetylase inhibitors such as suberoylanilide hydroxamic acid (SAHA), trichostatin A (TSA), and NaPB have been suggested to protect neurons in HD by promoting the intracellular transport of BDNF through the inhibition of HDAC6 and subsequent tubulin acetylation. Furthermore, HDAC inhibitors were suggested more generally to find use for the treatment of neurodegenerative diseases associated with altered intracellular transport such as Alzheimer's disease (Dompierre et al., 2007).

Accordingly, the other active substance may be a HDAC inhibitor preferably where the neurodegenerative disorder is HD or Alzheimer's disease.

Where it is contemplated to induce β-TrCP overexpression to restore β-catenin degradation, the other active substance may be, e.g., a calcineurin inhibitor, such as FK506.

### Screening methods

The results disclosed herein strongly suggest that Htt would be a scaffold protein which facilitates β-catenin interaction with β-TrCP and association with the destruction complex. This scaffold function would be impaired in polyQ-Htt thereby resulting in loss of β-catenin / β-TrCP interaction.

Accordingly, it is proposed that molecules capable of restoring normal interaction of polyQ-Htt with β-catenin that could prevent β-catenin accumulation and neuronal death in Huntington's disease, e.g. by inducing post-translational modifications of polyQ-Htt.

A method for screening molecules likely to prevent and/or treat Huntington's disease, which method comprises:
a) contacting a candidate molecule with a cell expressing polyglutamine-huntingtin (polyQ-Htt) and β-catenin; and
b) detecting if :
   i) polyQ-Htt interacts with β-catenin in the presence of said candidate molecule wherein, detection of an interaction between polyQ-Htt and β-catenin in the presence of said candidate molecule is indicative of a candidate molecule likely to prevent and/or treat Huntington's disease; and/or
   ii) β-catenin cellular level is decreased in the presence of said candidate molecule as compared with β-catenin cellular level in the absence of said candidate molecule, wherein decreased β-catenin cellular level is indicative of a candidate molecule likely to prevent and/or treat Huntington's disease; and/or
   iii) β-catenin localization at the cell membrane is increased in the presence of said candidate molecule as compared with β-catenin localization at the cell membrane in the absence of said candidate molecule, wherein increased β-catenin localization at the cell membrane is indicative of a candidate molecule likely to prevent and/or treat Huntington's disease; and/or
   iv) β-catenin degradation is increased in the presence of said candidate molecule as compared with β-catenin degradation in the absence of said candidate molecule, wherein increased β-catenin degradation is indicative of a candidate molecule likely to prevent and/or treat Huntington's disease.

The cell may have been transformed to express polyglutamine-huntingtin (polyQ-Htt).

The cell may further express the proteins constitutive of the axin destruction complex. In such a case, it may be also assessed whether the candidate molecule is capable of restoring β-catenin degradation by the destruction complex.

Methods are known to the one skilled in the art which are suitable to determine interaction between two proteins. Reference may be made for instance to Biacore, fluorescence-based such as FRET (fluorescence resonance energy transfer), or bioluminescence-based such as BRET (Bioluminescence Resonance Energy Transfer).

Use may be made of flurorescence techniques and phenotypic screening methods to measure β-catenin accumulation and/or degradation and/or specific localization within the cell (plasma membrane) as a read-out upon expression of polyQ-htt in cells and/or neurons.

The invention will be further illustrated in view of the following figures and examples.

### FIGURES

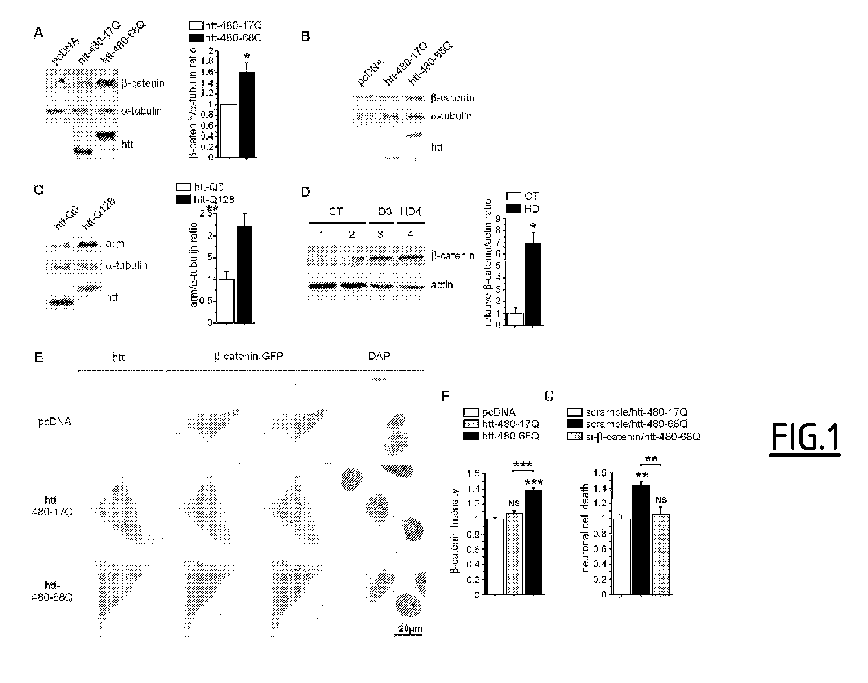

**Fig. 1**. Accumulation of β-catenin is toxic in HD. (*A* and *B*) β-catenin accumulates in cells expressing polyQ-huntingtin. MDCK (*A*) and HEK (*B*) cells are transfected with htt-480-17Q or htt-480-68Q constructs or empty vector (pcDNA) and analyzed by immunoblotting for the presence of β-catenin, α-tubulinand huntingtin. Quantification of 4 independent experiments in MDCK shows a statistically significant increase in β-catenin level. (*C*) Armadillo is stabilized in a *Drosophila* model of HD. UAS-htt^{548aa}-QO and UAS-htt^{548aa}-Q128 flies are crossed with *w;gmr-*Gal4;UAS-LacZ. Armadillo (arm) levels are analyzed in the progeny by immunoblotting. Quantification of 4 experiments reveals a strong increase of armadillo expression. (*D*) β-catenin is upregulated in HD brains. Protein extracts are prepared from *post-mortem* striatum of CT (samples 1 and 2) and HD individuals (HD grade 3, sample 3; HD grade 4, sample 4) and analyzed by immunoblotting for β-catenin and β-actin. Quantification of the Western Blot shows a statistically significant increase in the protein level of β-catenin in HD samples compared with control samples. Results are expressed as a ratio between β-catenin and α-tubulinor actin intensity. (*E*) PolyQ-huntingtin induces β-catenin relocalization. HeLa cells are transfected with β-catenin-GFP and htt-480-17Q, htt-480-68Q or the empty vector (pcDNA), fixed and immunostained for huntingtin and GFP. (*F*) Quantitative analysis of β-catenin localization expressed as a ratio of cytoplasm/nuclear intensities normalized to the control situation (pcDNA). (*G*) Preventing accumulation of β-catenin reduces toxicity in neurons. Striatal neurons were nucleofected with siRNA directed against β-catenin (si-β-catenin) or scramble RNA and 48 h later transfected with htt-480-17Q or htt-480-68Q. Data from 4 independent experiments demonstrate a protective effect of β-catenin downregulation towards polyQ-huntingtin-induced toxicity. (NS, not significant; *P<0.05; **P<0.01; ***P<0.001; see supporting information for detailed statistical analyses and number of measures).

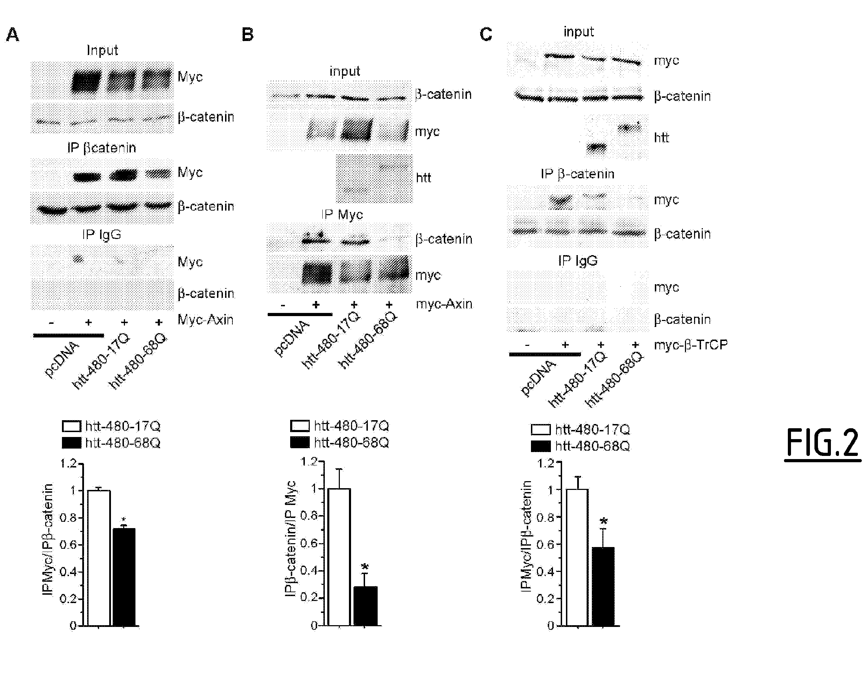

**Fig. 2**. The β-catenin destruction complex is altered in HD. (*A* and *B*) β-catenin binding to axin is impaired in polyQ context. HEK cells were transfected with myc-tagged axin and htt-480-17Q or htt-480-68Q constructs or empty vector (pcDNA). Immunoprcipitation experiments using β-catenin (*A*- IP β-catenin) or myc antibodies (*B* - IP Myc) revealed a strong decrease of β-catenin interaction with axin in presence of htt-polyQ compaired to wild-type htt. (C) PolyQ expansion affects β-catenin interaction with β-TrCP. β-catenin immunoprecipitation in HEK cells expressing both myc-β-TrCP and a fragment of htt (htt-480-17Q or -68Q) show a defect of β-catenin and β-TrCP binding in cells expressing polyQ-htt. The graph represent the quantitative assessment of the optical densities of the co-immunoprecipitated versus immunoprecipitated proteins. (*P<0.05, see supporting information for detailed statistical analyses and number of measures).

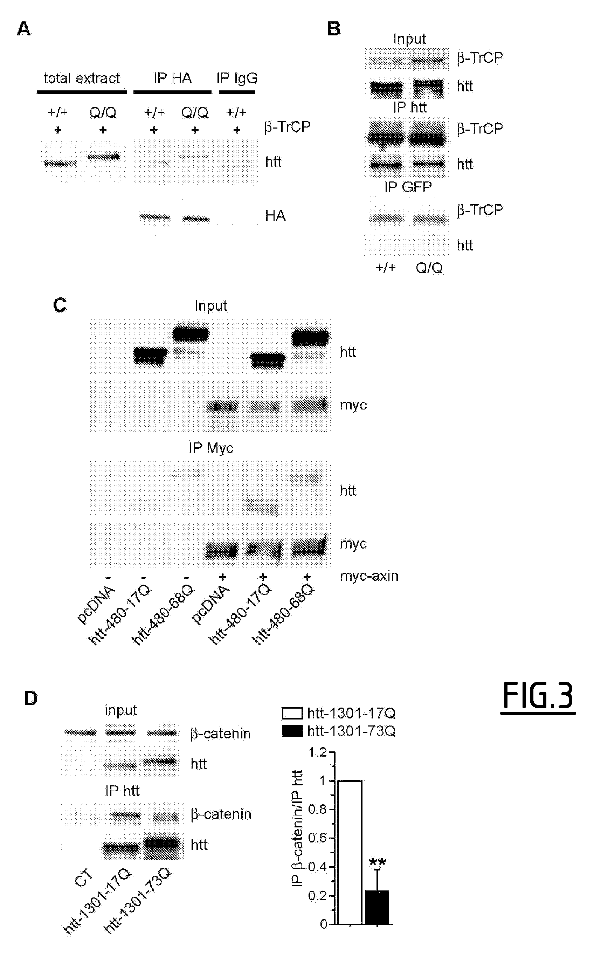

**Fig. 3****.** Htt-polyQ modulates β-catenin binding to the destruction complex. (*A*) Huntingtin and β-TrCP interact in a polyQ independent manner. Primary culture of cortical neurons from wild-type mice (+/+) or *Hdh*^{Q109/Q109} knock-in mice (Q/Q) are electroporated with HA tagged β-TrCP. β-TrCP immunoprecipitation (HA antibody; IP HA) experiments show an interaction of β-TrCP with endogenous huntingtin. No difference in the interaction is observed between wild-type and polyQ-huntingtin. A mouse IgG is used as a control. (*B*) Equivalent results are obtained doing the converse immunoprecipitation (4C8; IP htt) on whole brain extracts from wild-type mice (+/+) or *Hdh*^{Q109/Q109} knock-in mice (Q/Q). Anti-GFP antibody is used to perform the control immunoprecipitation. (*C*) Htt and axin interact in a polyQ independent manner. HEK 293 cells are transfected with htt-480 constructs or the corresponding empty vector (pcDNA) and myc-axin. A myc immunoprecipitation reveals that htt interacts with axin and that this binding is not modified by the polyQ-expansion. (*D*) PolyQ expansion alters huntingtin binding to β-catenin. HEK 293 cells are transfected with different YFP-fused htt-1301 variants and the corresponding empty vector (control, CT). A 4C8 immunoprecipitation shows a decreased interaction of huntingtin with β-catenin in polyQ-huntingtin compared to wild-type huntingtin expressing cells. The graph represents the quantitative assessment of the optical densities of β-catenin versus huntingtin immunoprecipitates. (*P<0.05, **P<0.01; see supporting information for detailed statistical analyses and number of measures).

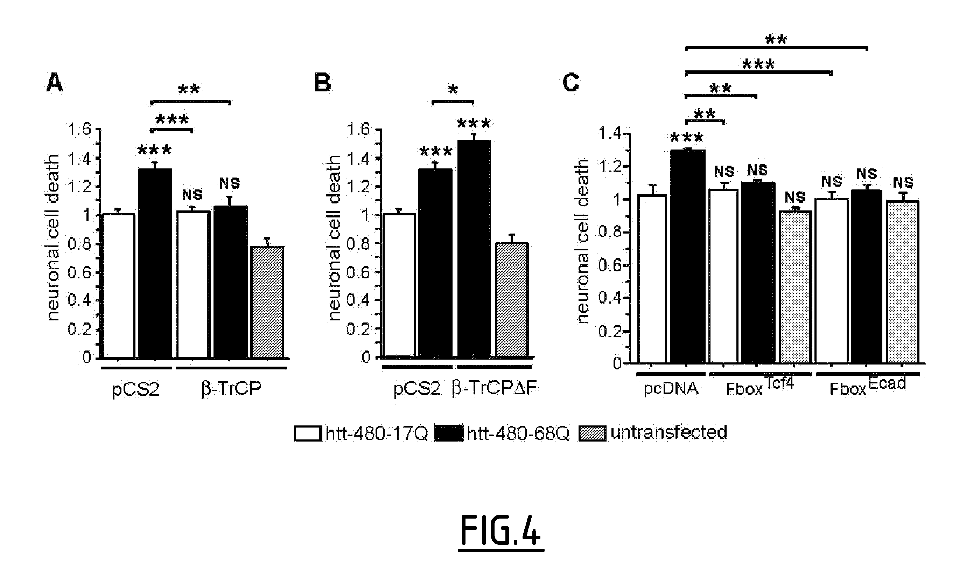

**Fig. 4****.** β-TrCP restores polyQ-induced toxicity in striatal neurons. Wild-type huntingtin (htt-480-17Q) and polyQ-huntingtin (htt-480-68Q) are co-transfected with β-TrCP, the dominant negative form β-TrCPΔF or the empty vector (pCS2) in striatal neurons. Data from six independent experiments reveal that β-TrCP significantly reduces polyQ-huntingtin-induced cell death (*A*), whereas β-TrCPΔF has the opposite effect (*B*). (*C*) Neuronal cell death was assessed in primary culture of striatal neurons transfected with htt constructs (htt-480-17Q or 68Q) and chimeric F-box fusion proteins, Fbox-BBD^{Tcf4} or Fbox-βBD^{Ecad}. Data from 4 independent experiments show a significant reduction of polyQ-htt-induced toxicity in cells expressing Fbox proteins that can only degrade β-catenin. (NS, not significant; *P<0.05; **P<0.01; ***P<0.001; see supporting information for detailed statistical analyses and number of measures).

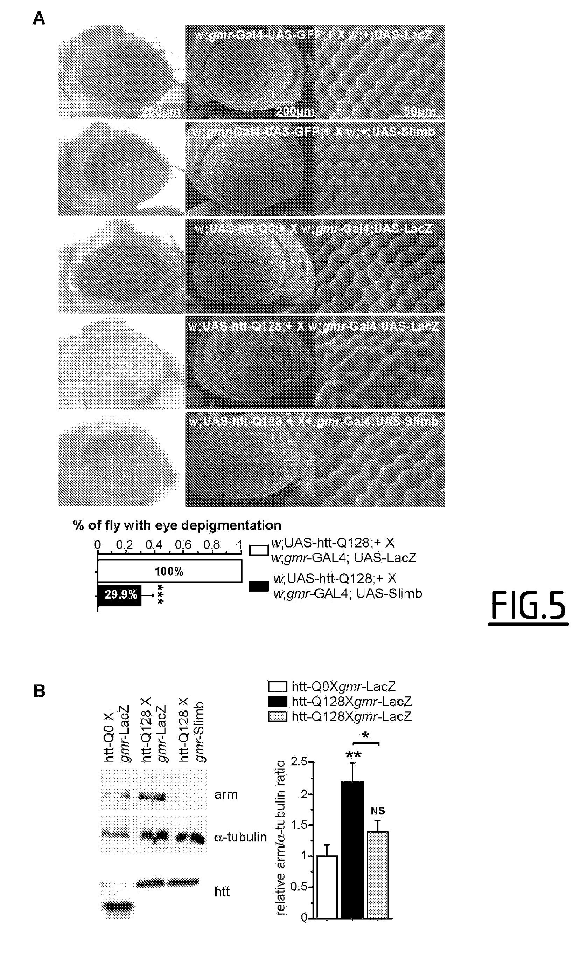

**Fig. 5****.** Slimb, the *Drosophila* homologue of β-TrCP, reduces polyQ-induced toxicity and β-catenin level in a *Drosophila* model of HD. (*A*) Left panel: Slimb regulates polyQ-huntingtin-induced eye depigmentation. The following crosses are analyzed : *w*;*gmr*-Gal4-UAS-GFP with *w*;+;UAS-LacZ or with *w*;+;UAS-Slimb as controls; UAS-htt^{548aa}-Q0 with *w*;gmr-Gal4;UAS-LacZ and UAS-htt^{548aa}-Q128 with *w;*gmr-Gal4;UAS-LacZ or *w*;gmr-Gal4;UAS-Slimb. The progeny (females) is analyzed at 10 days for eye pigmentation. Eye-specific polyQ-huntingtin expression leads to a complete loss of pigmentation (compare line 4 to line 3). Expression of UAS-Slimb transgene in those flies rescue the toxicity (line 5). The graph represents the percentage of flies with more than 50% of eye depigmentation. Right panels: Visualization of ommatidial morphology using scanning electron microscopy. PolyQ-huntingtin expression induces a strong desorganisation of ommatidia that is compensated by expression of slimb. (*B*) Slimb reverses β-catenin accumulation in polyQ-huntingtin expressing *Drosophila.* UAS-htt^{548aa}-Q0 and UAS-Htt^{548aa}-Q128 flies are crossed with *w*;gmr-Gal4;UAS-LacZ or *w*;gmr-Ga14;UAS-Slimb. Immunoblotting analysis of the progeny shows a loss of β-catenin accumulation in htt-548-Q128 flies expressing Slimb as compared to lacZ. Quantification of 4 independent experiments shows an important rescue of β-catenin level when slimb is expressed. Results are expressed as a ratio between armadillo and α-tubulinsignal intensity and are normalized to control (htt-Q0 X *gmr*-LacZ). (NS, not significant; *P<0.05; **P<0.01; ***P<0.001; see supporting information for detailed statistical analyses and number of measures).

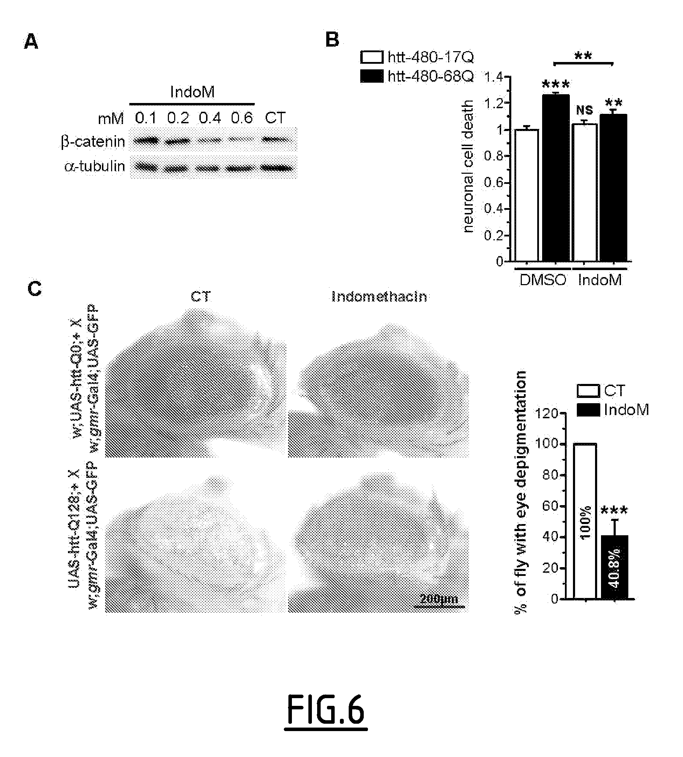

**Fig. 6****.** Indomethacin reduces htt-polyQ-induced toxicity in neurons and in *Drosophila.* (*A*) Indomethacin treatment of striatal neurons reduces the level of β-catenin as revealed by immunoblotting. (*B*) Primary cultures of striatal neurons are transfected with htt-480-17Q or htt-480-68Q and treated with indomethacin (indoM, 600µM) or 1% DMSO for 24 hours. Indomethacin treatment results in a statistically significant decrease of polyQ-htt-induced neuronal death. (C) Wild-type htt-548-Q0 and polyQ htt-548-Q128 flies are feed with indomethacin. Expressing mutant polyQ-huntingtin in developing eyes leads to adults with a loss of eye pigmentation. Indomethacin rescues the loss of eye pigmentation induced by mutant huntingtin in the developing eyes of flies in a statistically significant manner. (**P<0.01; ***P<0.001; see supporting information for detailed statistical analyses and number of measures).

### EXAMPLES

### Example 1: Materials and methods

**Statistical analyses.** Statview 4.5 software (SAS Institute, Cary, NC) was used for statistical analyses. All data herein described were performed in triplicate. Data are expressed as means ± SEM. Statistical analyses may be found in the supporting information.

**DNA constructs, siRNA and antibodies.** Constructs encoding β-galactosidase, β-catenin-enhanced green fluorescent protein (eGFP), Myc- or hemagglutinin (HA)-tagged β-TrCP, inactive HA-β-TrCPΔF and FLAG-Fbox-βBD^{Tcf4} and FLAG-Fbox-βBD^{Ecad} were described elsewhere (Delmas et al., 2007; Margottin et al., 1998, Liu, 2004). The wild-type and polyQ-huntingtin constructs htt-480-17Q, htt-480-68Q, YFP-htt-1301-17Q and YFP-htt-1301-73Q were previously described (Anne et al., 2007; Saudou et al., 1998). The siRNA sequence targeting rat β-catenin was already described (Gu et al., 2008). The scramble RNA (scRNA, control; Eurogentec, Seraing, Belgium) used had a unique sequence which did not match with any sequence in the genome of interest. The following antibodies were used: mouse monoclonal anti-htt (clone 1HU-4C8; (Trottier et al., 1995), WB 1:5000, IF 1:200), anti-β-catenin (BD bioscience, San Jose, CA, WB 1:5000), anti-a-tubulin(clone DM1A, Sigma, St. Louis, MO, WB, 1:3000), anti-β-galactosidase (Promega, Madison, WI, IF, 1:100), anti-β-TrCP (clone 1B1D2; Zymed, San Fransisco, CA, WB:1:500) , anti-β-actin (Sigma, St. Louis, MO, WB, 1:5000), anti-GFP (Roche, Mannheim, Germany), anti-Armadillo (clone N27A1, ascites, Developmental Studies Hybridoma Bank, Iowa city, IA, WB 1:500) and anti-mouse IgG (Upstate, Charlottesville, VA, USA), polyclonal rabbit anti-GFP (Millipore, Bedford, MA, IF 1:200) and monoclonal Rat anti-HA (clone 3F10, Roche, Mannheim, Germany, WB 1:5000). Anti-mouse and anti-rabbit secondary antibodies conjugated to AlexaFluor-488, AlexaFluor-555 were purchased from Molecular Probes (Invitrogen, Breda, The Netherlands). Anti-mouse and anti-rat secondary antibodies conjugated to HRP were purchased respectively from Jackson Immunoresearch Laboratories (West Grove, PA) and Beckman coulter (Fullerton, CA). Indomethacin was purchased from Sigma (Sigma, St. Louis).

**Cell culture, transfection.** Primary cultures of striatal neurons were prepared from embryonic day 17 Sprague Dawley rats (Saudou et al., 1998) and transfected at 4 days *in vitro* by a modified calcium phosphate technique (Xia et al., 1996) or by lipofectamine (Invitrogen, Carlsbad, CA). Primary cultures of cortical neurons were prepared from knock-in wild-type *Hdh*^{Q7/Q7} and mutant *Hdh*^{Q109/Q109} mice (Wheeler et al., 2002; Wheeler et al., 2000) at embryonic day 14. When indicated, neurons were electroporated the day of plating with the rat neuron Nucleofector kit (Amaxa Biosystems, Cologne, Germany). Human embryonic kidney 293 (HEK293), Madin-Darby canine kidney (MDCK) and human epithelial HeLa cells were cultured in DMEM supplemented with 10% bovine calf serum (BCS) and 6 mM L-glutamine. HEK cells were transfected by the calcium phosphate technique whereas MDCK and HeLa cells were transfected by lipofectamine (Invitrogen, Carlsbad, CA).

**Cell extracts, immunobloting and immunoprecipitation experiments.** HEK and MDCK cells were lyzed 48 h after transfection, in Triton X-1 00 buffer (pH 7.4; 160 mM NaCl; 50 mM Hepes; 2.5 mM MgCl₂; 1.5 mM CaCl₂; 2.5 mM KCI; 1% Triton; 1 mM PMSF; 2 µg/ml Pepstatin; 2 µg/ml leupeptin; 2 µg/ml Aprotinin; 1 mM lodoacetamide; 2 mM DTT; and 1 mM orthovanadate) and centrifuged at 11,000 x g (10 min; 4°C). Proteins (1-20 µg) were loaded onto SDS-PAGE and subjected to Western blot analysis.

For immunoprecipitation with anti-huntingtin (4C8) antibody, HEK transfected cells or brain from knock-in wild-type *Hdh*^{Q7/Q7} and mutant *Hdh*^{Q109/Q109} mice (Wheeler et al., 2002; Wheeler et al., 2000) were lyzed in 1% Triton X-100 buffer containing protease and phosphatase inhibitors and centrifuged at 16,000 g for 15 min. Lysates (500 µg at 1 µg/µl) were precleared 1 h at 4°C with 50 µl of a 50% solution of protein G beads. Extracts were incubated for 1 h at 4°C with 2 µl of anti-huntingtin (4C8) antibody or 4 µl of anti-GFP prebound with 50 µl of a 50% solution of protein G sepharose beads (Sigma). Beads were washed three times with Triton X-100 buffer. For immunoprecipitation with anti-HA antibody, primary culture of cortical neurons from KI mice were lysed in 1% Triton X-100 buffer containing protease and phosphatase inhibitors and centrifuged at 16,000 g for 15 min. Lysates (300 µg at 1 µg/µl) were incubated 1 h at 4°C with 2 µl of anti-HA antibody or 8µl of mouse-IgG and 2 h with 40 µl of protein G sepharose beads (50 % solution). Beads were washed three times with Triton X-100 buffer. Bound proteins were eluted with sodium dodecyl sulfate (SDS) loading buffer and resolved by SDS-PAGE (polyacrylamide gel electrophoresis) and subjected to immunoblotting analysis. For immunoprecipitation with anti-β-catenin antibody, HEK cells were lyzed in 0.5% Triton X-100 buffer (pH 7.5, 150mM NaCl, 20mM Tris, 2mM EDTA, 10mM NaF, 10mM NaPPi, 50mM KH₂PO₄, 0,5% Triton) containing protease and phosphatase inhibitors and centrifuged at 16,000 g for 15 min. Lysates (400 µg at 1 µg/µl) were precleared 1 h at 4°C with 25 µl of a 50% solution of protein G beads. Extracts were incubated overnight at 4°C with 2 µl of anti-β-catenin antibody or same amount of anti-IgG and 1 h with 40 µl of protein G sepharose beads (50 % solution). Beads were washed three times with lysis buffer. Analysis of interaction between β-catenin and axin or htt using anti-Myc antibody were done respectively in 0.5% Triton X-100 buffer and in 0.1% Triton X-100 buffer (pH 7.5, 150mM NaCl, 20mM Tris, 400µM EDTA, 10mM NaF, 10mM NaPPi, 0.1 % Triton). Lysates (400 µg at 1 µg/µl) were precleared 1 h at 4°C with 25 µl of a 50% solution of protein G beads. Extracts were then incubated for 1 hour at 4°C with 2 µl of anti-myc antibody and 1 h with 40 µl of protein G sepharose beads (50 % solution). Beads were finally washed three times with lysis buffer.

**Brain tissues.** Tissues were obtained from the Harvard Brain Tissue Resource Center (HBTRC; Belmont, MA): two controls (samples 1-2), one HD grade 3 (HD3, sample 3), and one grade 4 (HD4, sample 4) patients. Samples correspond respectively to brain numbers 4741, 4744, 4797 and 4680 as numbered by HBTRC. Samples were homogenized in NP-40 lysis buffer and cleared by centrifugation at 6000 x *g* (15 min; 4°C). Western blot analysis was performed on 10 µg of total extracts.

**Immunofluorescence experiments.** HeLa cells were grown on uncoated glass coverslips and transfected with β-catenin-eGFP and htt-480-17Q, htt-480-68Q or empty vector (pcDNA) (ratio 7:1) for 48 h. Cells were fixed with 4% paraformaldehyde (PFA) for 20 min and incubated with anti-GFP (rabbit, 1:200) and anti-Htt (4C8; 1:200) antibodies. Pictures were collected using a 3D microscope (Gauthier et al., 2004) or a confocal Leica SP5. Projections, and analyses were generated using ImageJ software (Rasband, W.S., ImageJ, U. S. National Institutes of Health, Bethesda, Maryland, USA, http://rsb.info.nih.gov/ij/, 1997-2009). For β-catenin-GFP quantifications, a home-built macro was used (A. Chapelle and O. Anezo). GFP average intensity was measured in a 10 pixel wide square in the nucleus and in the cytoplasm. The background was also evaluated by measuring the intensity outside the cell. This value was subtracted to cytoplasm and nucleus intensities. We analyzed the ratio between cytoplasm and nucleus intensity. A minimum of 250 cells per condition was scored.

**Drosophila Stocks and experiments.** UAS-LacZ (on III) and *gmr*-Gal4 (on II) and *gmr*-Gal4-UAS-GFP (on II) strains were obtained from F. Maschat; UAS-Htt^{548aa}-Q0 and UAS-Htt^{548aa}-128Q from J. Troy Littleton (Lee et al., 2004) and UAS-Slimb (on III) from B. Limbourg-Bouchon (Grima et al., 2002). Using those stocks, we generated the following homozygous strains: *w*;gmr-Gal4;UAS-LacZ and *w*;gmr-Gal4;UAS-Slimb. All flies were raised on standard medium at 25 °C.

For rescue experiments, we crossed either virgin w; UAS-Htt^{548aa}-Q0 or w; UAS-Htt^{548aa}-Q128 with *w*;gmr-Gal4;UAS-Slimb or *w*;gmr-Gal4;UAS-LacZ males. As control, virgins *w*;gmr-Gal4-UAS-GFP were crossed with UAS-LacZ and UAS-Slimb males. All crosses were done at the same time and under the same condition: flies were allowed to mate for 3 days at 25°C, and then the eggs were transferred to 29°C. Phenotypes were analyzed between 10 and 12 days after adults eclosion, when the eye depigmentation is maximal for UAS-Htt^{548aa}-128Q X *w*;gmr-Gal4;UAS-LacZ crosses. As a rescue, we considered fly with over than 50% of remaining pigmentation. For analysis of ommatidial morphology, flies were frozen on dry ice, and kept at -80°C until used. Before use, all samples were dehydrated and coated with gold/palladium. Structure of ommatidia was then analyzed by scanning electron microscopy. For Western Blotting experiments, crosses were performed at 25°C and one day old adult were collected. *Drosophila* were frozen in dry ice, heads were isolated and homogenized in lysis buffer (25 mM Tris-HCl, 5 mM EDTA, 250 mM NaCl and 1% triton supplemented with protease and phosphatases inhibitors cocktail (Sigma, Steinhelm, Germany)), let for 20 min on ice and centrifuged at 11,000 x g for 20 min at 4°C. Equal amount of proteins were loaded onto SDS-PAGE and subjected to Western blot analysis.

For indomethacin rescue experiment virgins *w*;gmr-Ga14-UAS-GFP were crossed with UAS-Htt^{548aa}-Q0 or w; UAS-Htt^{548aa}-Q128 males on standard drosophila food containing either indomethacin (250mg/L) or EtOH as control. Flies were allowed to mate for 3 days at 25°C, and then the eggs were transferred to 29°C. Adult were isolated everyday and transferred to a fresh tube every other day. Phenotypic analyses were conducted as described above.

**Measurement of neuronal survival.** Three days after plating, primary cultures of striatal neurons were transfected by a modified calcium phosphate technique (Saudou et al., 1998) with the same amount of wild-type or polyQ-huntingtin and various β-TrCP plasmids. Cytomegalovirus-β-galactosidase plasmid (10:1 ratio) was also added to identify the transfected cells. Forskolin (10 µM; Sigma) and IBMX (100 µM; Sigma) were added to the cultures 2 h after transfection. One day after transfection, cells were fixed with 4% paraformaldehyde (PFA) for 20 min and immunostained with anti-htt 4C8 and anti-β-galactosidase antibodies. When stated, neurons were electroporated the day of plating with si-β-catenin or scramble RNA and, 2 days after electroporation, transfected by lipofectamine (Invitrogen, Carlsbad, CA) with the different huntingtin constructs. For indomethacin experiments, cells were transfected with lipofectamine 2 days after plating. Conditioned media containing either indomethacin or DMSO was added to cells 6 hours after transfection for 24 hours. Cells were fixed and immunostained with anti-huntingtin 4C8 antibody 1 day after transfection. 4C8-positive neurons of similar intensities were scored under fluorescence microscopy in a blinded manner. Neuronal degeneration was assessed by neurite loss and nuclear shrinkage (Anne et al., 2007). Cell death was expressed as a fold increase in neuronal cell death relative to the death induced by the htt-480-17Q construct. Each graph represents four to six independent experiments performed in duplicate. Each bar in a given graph corresponds to the scoring of approximately 2000 neurons. Data were submitted to complete statistical analysis. β

### Example 2: β-catenin accumulates in HD.

To analyze the effect of polyQ-huntingtin on β-catenin, we expressed N-terminal fragments of huntingtin that contain the first 480 amino acids with 17Q (wild-type, htt-480-17Q) or 68Q (mutant, polyQ, htt-480-68Q) in MDCK and HEK 293 cells and determined the level of β-catenin by immunoblotting (Fig. 1*A* and *B*). Whereas wild-type huntingtin had no effect on β-catenin expression, expression of the htt-480-68Q fragment resulted in a significant increase in total β-catenin in MDCK and HEK 293 cells.

We next wondered whether this accumulation of β-catenin occured *in vivo.* We used a *Drosophila* model, where the N-terminal 548 amino acids of human huntingtin containing 0 (htt-548-Q0) or 128 (htt-548-Q128) glutamines, is inserted into the genome under the control of upstream activator sequences (UAS) that are recognized by the Gal4 transcription activator (Lee et al., 2004). This model recapitulates different hallmarks of HD (Lee et al., 2004). Htt-548-Q0 and htt-548-Q128 forms of huntingtin were expressed by using the eye-specific *gmr-*Gal4 driver, and the amount of armadillo (arm), the *Drosophila* homologue of β-catenin, was analysed by immunobloting (Fig. 1 C). We found that armadillo levels were two fold increased in the htt-548-Q128 flies compared to htt-548-Q0 flies.

To confirm the physiological relevance of this accumulation in HD, we assessed the level of β-catenin in *post-mortem* striatal samples from grade 3 and 4 HD patients by Western Blot analyses (Fig. 1 D) and observed a dramatic increase of β-catenin in HD patients compared to control individuals. Together, these results establish that β-catenin accumulates in cellular and *Drosophila* models of HD as well as in *post-mortem* samples from patients.

It has been well demonstrated that when not degraded, β-catenin accumulates in the cytoplasm and then diffuses to the nucleus. We thus examined whether polyQ-huntingtin could influence the intracellular localization of β-catenin (Fig. 1*E*). For this purpose, we investigated the subcellular localisation of GFP-tagged β-catenin constructs in HeLa cells expressing either empty vector (pcDNA) or huntingtin constructs (htt-480-17Q or -68Q). As expected the overexpressed β-catenin localizes mainly to the nucleus in control cells and in cells expressing wild-type huntingtin. Surprisingly, GFP-β-catenin localized both in the cytoplasm and in the nucleus in cells transfected with htt-480-68Q (Fig. 1 E). Moreover the ratio between cytoplasmic and nuclear β-catenin was significantly increased in cells expressing polyQ-huntingtin as compared to control or wild-type huntingtin expressing cells (Fig. 1 F). Together these results show that mutant huntingtin overexpression results in β-catenin accumulation in the cytoplasm.

### Example 3: Accumulation of β-catenin is toxic in HD.

To establish whether the accumulation of β-catenin observed in HD could promote neuronal toxicity, we tested whether reduction of β-catenin expression could influence the polyQ-induced toxicity by studying a neuronal model of HD that recapitulates several features of the disease (Saudou et al., 1998). Primary culture of striatal neurons, were first electroporated with a siRNA directed against β-catenin or with a scramble RNA and then transfected with htt-480-17Q or htt-480-68Q constructs (Fig. 1 *G*). As expected, the 480-68Q fragment induced a statistically significant increase in neuronal death compared with the htt-480-17Q construct under control condition (scramble RNA) (Fig. 1*G*). Interestingly, downregulation of β-catenin level completely inhibited polyQ-huntingtin-induced toxicity, suggesting that the abnormal accumulation of β-catenin in HD is toxic.

### Example 4: The β-catenin destruction complex is altered in HD.

The levels of β-catenin are tightly regulated in cells that constantly synthesize and degrade β-catenin. In particular, the destruction complex is required to continually process the synthesized β-catenin by generating ubiquitinated β-catenin. As β-catenin accumulates in HD, we asked what are the consequences of the abnormal polyQ expansion in mutant huntingtin on the formation of the β-catenin destruction complex. We first assessed whether polyQ-htt could modulate recruitment of β-catenin to the destruction complex. We thus analyzed the interaction between β-catenin and axin, a core component of the destruction complex. We performed immunoprecipitation experiments using anti-myc and anti-β-catenin antibodies from HEK cells extracts expressing HA-tagged axin and htt-480-17Q or htt-480-68Q. The binding of β-catenin to axin was significantly decreased in polyQ context suggesting that β-catenin binding to the degradation complex is impaired in HD (Fig. 2A and *B*). As β-catenin degradation requires binding to the SCF^{β-TrCP} E3 ubiquitin ligase complex, we also asked whether mutant htt influence the interaction between β-catenin and the F-box protein β-TrCP, the substrate-recognizing subunits of the SCF complex. In HEK cells expressing β-TrCP-Myc and wild-type or polyQ-htt, we performed β-catenin immunoprecipitation experiments and found that the interaction between β-catenin and β-TrCP was reduced in presence of mutant htt (Fig. 2C). Thus, the presence of polyQ-htt impairs the association of β-catenin both with the core of the destruction complex and with the ubiquitination activity-containing complex.

### Example 5: PolyQ-htt regulates the interaction between β-catenin and β-TrCP.

We then examined whether huntingtin interact with β-catenin, β-TrCP and axin and what would be the consequence of the abnormal polyQ expansion in mutant huntingtin. We used primary cultures of striatal neurons from wild-type mice or *Hdh*^{109Q/109Q} knock-in mice (Wheeler et al., 2002; Wheeler et al., 2000), in which a CAG expansion is inserted into the endogenous mouse huntingtin gene. These neurons were electroporated with β-TrCP-HA and we performed immunoprecipitation (IP) experiments using an anti-HA antibody. β-TrCP bound to endogenous huntingtin (Fig. 3A). Moreover those proteins interacted at endogenous level as demonstrated by anti-huntingtin IP experiments from wild-type and *Hdh*^{109Q/109Q} mice brain extracts (Fig. 3*B*). The polyQ expansion did not influence the interaction of huntingtin with β-TrCP as no obvious differences were observed in anti-HA and anti-huntingtin IP experiments in wild-type and *Hdh*^{109Q/109Q} conditions (Fig. 3A and *B*). We next studied the interaction between htt and axin in HEK cells transfected with myc-tagged axin and fragment of htt ((htt-480-17Q or -68Q) or empty vector (pcDNA) using anti-myc antibody. We demonstrated that htt interacted with axin and that this interaction is not modulated by the polyQ expansion (Fig. 3C). We finally analysed huntingtin binding to β-catenin in HEK 293 cells transfected with the 1301 amino acid N-terminal fragments of huntingtin containing 17 (wild-type, htt-1301-17Q) or 73 (polyQ, htt-1301-73Q) glutamines. We used these 1301 amino acid N-terminal fragments as they immunoprecipitated efficiently from HEK 293 cell extracts. Huntingtin bound β-catenin and this interaction was impaired in polyQ context, as the β-catenin co-immunoprecipitated fraction was decreased in cells transfected with htt-1301-73Q (Fig. 3D). Our results show that huntingtin form a complex with axin, β-catenin and β-TrCP, and that the interaction with β-catenin but not with β-TrCP and axin is altered in HD context. Together our interactions studies show that the decreased interaction between β-catenin and polyQ-htt compared to the wild-type situation reduces the binding of β-catenin to the core of the destruction complex and to β-TrCP.

### Example 6: β-TrCP but not its dominant negative form protects striatal neurons from polyQ-htt- induced cell death.

As β-catenin accumulates in HD and its degradation complex is impaired, we next investigated whether β-TrCP possessed neuroprotective properties in HD. To this end, we evaluated the percentage of neuronal cell death in primary cultures of rat striatal neurons transfected with the 480-17Q and 480-68Q plasmids in the presence of a construct expressing HA-tagged β-TrCP or a HA-tagged dominant negative form lacking the F-Box (β-TrCPΔF). β-TrCPΔF is an inactive form of β-TrCP that cannot bind to the SCF complex subsequently blocking the degradation of phosphorylated β-catenin (Hart et al., 1999; Latres et al., 1999). Interestingly, β-TrCP decreased neuronal death induced by the htt-480-68Q fragment of huntingtin to levels comparable to the wild-type situation (Fig. 4A). However β-TrCPΔF, completely failed to rescue polyQ-huntingtin-induced neuronal death (Fig. 4*B*). These findings show that β-TrCP exerts a neuroprotective effect in a cellular model of HD and this depends on its ability to bind the E3 ubiquitin ligase complex SCF.

### Example 7: β-TrCP rescues neuronal cell death through the degradation of β-catenin.

To unequivocally address whether β-TrCP rescue polyQ-huntingtin-induced toxicity through the degradation of β-catenin, we analysed whether Fbox proteins that can only degrade β-catenin had neuroprotective properties in our cellular model of HD. We co-transfected primary cultures of striatal neurons with htt-480-17Q or htt-480-68Q constructs and with FLAG-tagged chimeric F-box fusion proteins, Fbox-BBD^{Tcf4} or Fbox-βBD^{Ecad}. In the latter constructs, the WD40-repeat of β-TrCP was replaced with the β-catenin binding domain from Tcf4 or E-cadherin (Liu et al., 2004). Fbox-βBD^{Tcf4} and Fbox-βBD^{Ecad} drastically increase β-catenin ubiquitination and turnover (Liu et al., 2004). We found that, whereas those chimeric Fbox proteins had no effect on neuronal toxicity by themselves, they significantly reduced polyQ-induced toxicity to levels comparable to the wild-type situation (Fig. 4C). We conclude that the neuroprotective properties of β-TrCP in HD specifically depend on its capacity to degrade β-catenin.

### Example 8: Slimb rescues neurodegeneration in a Drosophila HD model.

We next evaluated the protective effect of β-TrCP in a more physiological situation. For this purpose, we asked what could be the effect of Slimb, the *Drosophila* homologue of β-TrCP in HD flies. We determined the phenotypes induced by mutant polyQ-huntingtin expression (htt-548-Q128) in the absence or in the presence of Slimb (Fig. 5A). Using a *gmr*-Gal4 line, driving expression in the eyes, we observed that the expression of wild-type htt-548-Q0 did not show any adult eye phenotype. In contrast, expressing mutant polyQ-huntingtin (htt-548-Q128) in developing eyes led to adults with a loss of eye pigmentation (left panel) and a rough eye phenotype correlated with abnormal ommatidial array as detected by scanning electron microscopy (right panels) (Lee et al., 2004). This phenotype is related to a progressive neurodegeneration of eye retina with a loss of photoreceptors (Lee et al., 2004). Interestingly, expression of UAS-Slimb transgene in htt-548-Q128 flies rescued the eye depigmentation defect and improved the ommatidial morphology compared to htt-548-Q128 fly expressing neutral UAS transgene (UASLacZ). As a control, we ensured that Slimb transgene expression did not have a phenotype by itself by crossing UAS-Slimb with GMR-Gal4-UAS-GFP flies. We next quantified the rescue in loss of eye pigmentation. When the depigmentation was maximal in polyQ-huntingtin expressing flies (100% of flies), we calculated the percentage of flies expressing UAS-Slimb and htt-548-Q128 and showing a loss of eye pigmentation phenotype. We found that Slimb overexpression decreased over 60% the loss of pigmentation (Fig. 5A, graph). Together our results show that Slimb rescues the loss of eye pigmentation and the ommatidial morphology in *Drosophila,* two hallmarks of neuronal degeneration in this *in vivo* HD model.

Having shown that β-catenin accumulated in HD, we next tested whether the restoration of polyQ-induced toxicity was concomitant with rescue of β-catenin level. We analyzed the level of total armadillo by immunoblotting of extracts of *Drosophila* expressing wild-type or polyQ-huntingtin in the eye in the presence or absence of Slimb. When Slimb was overexpressed, armadillo failed to accumulate in htt-548-Q128 expressing fly (Fig. 5*B*). In our experimental condition, Slimb by itself did not modulate the level of armadillo expression (data not shown). In agreement, it has been previously shown that downregulation of armadillo by RNA interference suppress eye degenerescence in a drosophila model of HD (Kaltenbach et al., 2007). We also assessed the amount of polyQ-huntingtin in HD *Drosophila* expressing Slimb or not (Fig. 5*B*) and observed that those remained unmodified in both conditions. This shows that the neuroprotective effect of Slimb in HD is linked to its ability to degrade β-catenin/armadillo *in vivo.*

### Example 9: The anti-inflammatory drug indomethacin reduces htt-polyQ-induced toxicity in neurons and in Drosophila.

As indomethacin, an inhibitor of cyclooxygenase, was shown to downregulate β-catenin mRNA and protein level in cells (Dihlmann et al., 2001; Hawcroft et al., 2002; Kapitanovic et al., 2006), we tested whether this drug could have neuroprotective properties in a neuronal model of HD. By immunoblotting analyses, we first observed that indomethacin treatment of neurons reduced the level of β-catenin (Fig. 6A). Next, we transfected primary cultures of striatal neurons with wild-type or polyQ-htt (htt-480-17Q or htt-480-68Q) and treated these cells with indomethacin (600µM) or 1% DMSO for 24 hours. In agreement with a toxic role for the accumulation of β-catenin in HD, indomethacin treatment resulted in a reduced polyQ-htt-induced neuronal toxicity (Fig. 6*B*).

We next asked whether indomethacin could promote such a neuroprotective effect in a more physiological situation. For this purpose, we analyzed the effect of indomethacin on wild-type htt-548-Q0 and polyQ htt-548-Q128 flies. As shown previously (Fig. 6C), expressing mutant polyQ-huntingtin (htt-548-Q128) in developing eyes led to adults with a loss of eye pigmentation (left panel). Interestingly, this defect was rescued by indomethacin treatment. We quantified this effect when the depigmentation was maximal in polyQ-huntingtin expressing flies (100% of flies) and found that indomethacin led significantly reduced the loss of pigmentation (Fig. 6C, graph). Together our results show that indomethacin rescues degeneration in cells and in an *in vivo* HD model strongly supporting the notion that this drug could be a suitable HD treatment.

### References

Anne, S. L., Saudou, F., and Humbert, S. (2007). Phosphorylation of huntingtin by cyclin-dependent kinase 5 is induced by DNA damage and regulates wild-type and mutant huntingtin toxicity in neurons. J Neurosci 27, 7318-7328.
Carmichael J, Sugars KL, Bao YP, Rubinsztein DC. (2002) Glycogen synthase kinase-3beta inhibitors prevent cellular polyglutamine toxicity caused by the Huntington's disease mutation. J Biol Chem. 277(37):33791-8.
Delmas, V., Beermann, F., Martinozzi, S., Carreira, S., Ackermann, J., Kumasaka, M., Denat, L., Goodall, J., Luciani, F., Viros, A., et al. (2007). Beta-catenin induces immortalization of melanocytes by suppressing p161NK4a expression and cooperates with N-Ras in melanoma development. Genes Dev 21, 2923-2935.
Dihlmann, S., Siermann, A., and von Knebel Doeberitz, M. (2001). The nonsteroidal anti-inflammatory drugs aspirin and indomethacin attenuate beta-catenin/TCF-4 signaling. Oncogene 20, 645-653.
Dompierre JP, Godin JD, Charrin BC, Cordelières FP, King SJ, Humbert S, Saudou F. (2007) Histone deacetylase 6 inhibition compensates for the transport deficit in Huntington's disease by increasing tubulin acetylation. J Neurosci. 27(13):3571-83.
Gauthier, L. R., Charrin, B. C., Borrell-Pages, M., Dompierre, J. P., Rangone, H., Cordelieres, F. P., De Mey, J., MacDonald, M. E., Lessmann, V., Humbert, S., and Saudou, F. (2004). Huntingtin controls neurotrophic support and survival of neurons by enhancing BDNF vesicular transport along microtubules. Cell 118, 127-138.
Gines S, Ivanova E, Seong IS, Saura CA, MacDonald ME. (2003) Enhanced Akt signaling is an early pro-survival response that reflects N-methyl-D-aspartate receptor activation in Huntington's disease knock-in striatal cells. J Biol Chem. 278(50):50514-22.
Grima, B., Lamouroux, A., Chelot, E., Papin, C., Limbourg-Bouchon, B., and Rouyer, F. (2002). The F-box protein slimb controls the levels of clock proteins period and timeless. Nature 420, 178-182.
Gu, W., Wells, A. L., Pan, F., and Singer, R. H. (2008). Feedback regulation between zipcode binding protein 1 and beta-catenin mRNAs in breast cancer cells. Mol Cell Biol 28, 4963-4974.
Hart, M., Concordet, J. P., Lassot, I., Albert, I., del los Santos, R., Durand, H., Perret, C., Rubinfeld, B., Margottin, F., Benarous, R., and Polakis, P. (1999). The F-box protein beta-TrCP associates with phosphorylated beta-catenin and regulates its activity in the cell. Curr Biol 9, 207-210.
Hawcroft, G., D'Amico, M., Albanese, C., Markham, A. F., Pestell, R. G., and Hull, M. A. (2002). Indomethacin induces differential expression of beta-catenin, gamma-catenin and T-cell factor target genes in human colorectal cancer cells. Carcinogenesis 23, 107-114.
Kaltenbach, L. S., Romero, E., Becklin, R. R., Chettier, R., Bell, R., Phansalkar, A., Strand, A., Torcassi, C., Savage, J., Hurlburt, A., et al. (2007). Huntingtin interacting proteins are genetic modifiers of neurodegeneration. PLoS Genet 3, e82.
Kang DE, Soriano S, Xia X, Eberhart CG, De Strooper B, Zheng H, Koo EH. (2002) Presenilin couples the paired phosphorylation of beta-catenin independent of axin: implications for beta-catenin activation in tumorigenesis. Cell. 110(6):751-62.
Kapitanovic, S., Cacev, T., Antica, M., Kralj, M., Cavric, G., Pavelic, K., and Spaventi, R. (2006). Effect of indomethacin on E-cadherin and beta-catenin expression in HT-29 colon cancer cells. Exp Mol Pathol 80, 91-96.
Latres, E., Chiaur, D. S., and Pagano, M. (1999). The human F box protein beta-Trcp associates with the Cul1/Skp1 complex and regulates the stability of beta-catenin. Oncogene 18, 849-854.
Lee, W. C., Yoshihara, M., and Littleton, J. T. (2004). Cytoplasmic aggregates trap polyglutamine-containing proteins and block axonal transport in a Drosophila model of Huntington's disease. Proc Natl Acad Sci U S A 101, 3224-3229.
Lepourcelet M, Chen YN, France DS, Wang H, Crews P, Petersen F, Bruseo C, Wood AW, Shivdasani RA. (2004) Small-molecule antagonists of the oncogenic Tcf/beta-catenin protein complex. Cancer Cell. 5(1):91-102.
Liu, J., Stevens, J., Matsunami, N., and White, R. L. (2004). Targeted degradation of beta-catenin by chimeric F-box fusion proteins. Biochem Biophys Res Commun 313, 1023-1029.
Margottin, F., Bour, S. P., Durand, H., Selig, L., Benichou, S., Richard, V., Thomas, D., Strebel, K., and Benarous, R. (1998). A novel human WD protein, h-beta TrCp, that interacts with HIV-1 Vpu connects CD4 to the ER degradation pathway through an F-box motif. Mol Cell 1, 565-574.
Saudou, F., Finkbeiner, S., Devys, D., and Greenberg, M. E. (1998). Huntingtin acts in the nucleus to induce apoptosis but death does not correlate with the formation of intranuclear inclusions. Cell 95, 55-66. Soriano S, Kang DE, Fu M, Pestell R, Chevallier N, Zheng H, Koo EH. (2001) Presenilin 1 negatively regulates beta-catenin/T cell factor/lymphoid enhancer factor-1 signaling independently of beta-amyloid precursor protein and notch processing. J Cell Biol. 152(4):785-94.
Takemaru KI, Ohmitsu M, Li FQ. An oncogenic hub: beta-catenin as a molecular target for cancer therapeutics. (2008) Handb Exp Pharmacol. 186:261-84.
Trottier, Y., Devys, D., Imbert, G., Saudou, F., An, I., Lutz, Y., Weber, C., Agid, Y., Hirsch, E. C., and Mandel, J. L. (1995). Cellular localization of the Huntington's disease protein and discrimination of the normal and mutated form. Nat Genet 10, 104-110.
Wheeler, V. C., Gutekunst, C. A., Vrbanac, V., Lebel, L. A., Schilling, G., Hersch, S., Friedlander, R. M., Gusella, J. F., Vonsattel, J. P., Borchelt, D. R., and MacDonald, M. E. (2002). Early phenotypes that presage late-onset neurodegenerative disease allow testing of modifiers in Hdh CAG knock-in mice. Hum Mol Genet 11, 633-640.
Wheeler, V. C., White, J. K., Gutekunst, C. A., Vrbanac, V., Weaver, M., Li, X. J., Li, S. H., Yi, H., Vonsattel, J. P., Gusella, J. F., et al. (2000). Long glutamine tracts cause nuclear localization of a novel form of huntingtin in medium spiny striatal neurons in HdhQ92 and HdhQ111 knock- in mice. Hum Mol Genet 9, 503-513.
Wu G, Xu G, Schulman BA, Jeffrey PD, Harper JW, Pavletich NP. (2003) Structure of a beta-TrCP1-Skp1-beta-catenin complex: destruction motif binding and lysine specificity of the SCF(beta-TrCP1) ubiquitin ligase. Mol Cell.11(6):1445-56
Xia, Z., Dudek, H., Miranti, C. K., and Greenberg, M. E. (1996). Calcium influx via the NMDA receptor induces immediate early gene transcription by a MAP kinase/ERK-dependent mechanism. Journal of Neuroscience 16, 5425-5436.

## Claims

1. An antagonist of β-catenin for use for preventing and/or treating a neurodegenerative disorder.

2. The antagonist for the use according to claim 1, for interfering with cytoplasmic accumulation of β-catenin in a neuronal cell.

3. The antagonist for the use according to claim 1 or 2, wherein said antagonist inhibits β-catenin expression in a neuronal cell.

4. The antagonist for the use according to claim 1 or 2, wherein said antagonist increases β-catenin degradation in a neuronal cell.

5. The antagonist for the use according to claim 1 or 2, wherein said antagonist induces β-catenin relocalization at a neuronal cell membrane.

6. The antagonist for the use according to claim 1 or 2, wherein said antagonist interferes with β-catenin binding to a transcription factor of the T-cell factor (TCF)/lymphocyte enhancer factor (LEF) family or to a β-catenin co-activator.

7. The antagonist for the use according to any one of claims 1 to 6, wherein said antagonist is selected from the group consisting of non-steroidal antiinflammatory drugs (NSAIDs), Histone deacetylase (HDAC) inhibitors, inhibitors of cyclooxygenase (COX) enzymes, imatinib or a salt or ester thereof, endostatin, a β-catenin antisense oligonucleotide, a β-catenin siRNA, an antibody or aptamer directed against β-catenin and which blocks interaction of β-catenin with TCF/LEF, a vector containing a β-TrCP encoding sequence, a F-box chimera, vitamin A and retinoids, vitamin D, differentiation-inducing factors (DIFs), lithium, curcumin, flavonoids, and small molecule inhibitors of β-catenin binding to TCF/Lef or a β-catenin co-activator.

8. The antagonist for the use according to any one of claims 1 to 7, wherein said antagonist is selected from the group consisting of a β-catenin antisense oligonucleotide, a β-catenin siRNA, an antibody or aptamer directed against β-catenin and which blocks interaction of β-catenin with TCF/LEF, a vector containing a β-TrCP encoding sequence, and a F-box chimera.

9. The antagonist for the use according to any one of claims 1 to 7, wherein said antagonist is selected from the group consisting of indomethacin, sulindac, sulindac metabolite, naproxen, ketoprofen, ibuprofen, acetyl salicylic acid, NO-acetyl salicylic acid, celecoxib, rofecoxib, PKF115-854, CGP049090, ICG-001 and suberoylanilide hydroxamic acid (SAHA), trichostatin A (TSA), and NaPB.

10. The antagonist for the use according to any one of claims 1 to 9, wherein said neurodegenerative disorder is a disorder in which neuronal toxicity is associated with cytoplasmic β-catenin accumulation.

11. The antagonist for the use according to any one of claims 1 to 10, wherein said neurodegenerative disorder is selected from the group consisting of polyglutamine (polyQ) diseases, Alzheimer's disease, Parkinson's disease, prion diseases, multiple sclerosis, and amyotrophic lateral sclerosis.

12. The antagonist for the use according to any one of claims 1 to 11, wherein said neurodegenerative disorder is a polyglutamine (polyQ) disease selected from the group consisting of Huntington's disease, Kennedy disease, Haw River syndrome, Spinocerebellar ataxia type 1, Spinocerebellar ataxia type 2, Spinocerebellar ataxia type 3, Spinocerebellar ataxia type 6, Spinocerebellar ataxia type 7, Spinocerebellar ataxia type 12 and Spinocerebellar ataxia type 17.

13. The antagonist for the use according to any one of claims 1 to 12, wherein said neurodegenerative disorder is Huntington's disease.

14. The antagonist for the use according to any one of claims 1 to 13, wherein said antagonist is administered simultaneously or sequentially with another active substance.

15. A method for screening molecules likely to prevent and/or treat Huntington's disease, which method comprises:
a) contacting a candidate molecule with a cell expressing polyglutamine-huntingtin (polyQ-Htt) and β-catenin; and
b) detecting if :
i) polyQ-Htt interacts with β-catenin in the presence of said candidate molecule wherein, detection of an interaction between polyQ-Htt and β-catenin in the presence of said candidate molecule is indicative of a candidate molecule likely to prevent and/or treat Huntington's disease; and/or
ii) β-catenin cellular level is decreased in the presence of said candidate molecule as compared with β-catenin cellular level in the absence of said candidate molecule, wherein decreased β-catenin cellular level is indicative of a candidate molecule likely to prevent and/or treat Huntington's disease; and/or
iii) β-catenin localization at the cell membrane is increased in the presence of said candidate molecule as compared with β-catenin localization at the cell membrane in the absence of said candidate molecule, wherein increased β-catenin localization at the cell membrane is indicative of a candidate molecule likely to prevent and/or treat Huntington's disease; and/or
iv) β-catenin degradation is increased in the presence of said candidate molecule as compared with β-catenin degradation in the absence of said candidate molecule, wherein increased β-catenin degradation is indicative of a candidate molecule likely to prevent and/or treat Huntington's disease.
